# EUROPÄISCHE PATENTSCHRIFT

(11) **EP 3 484 537 B1**
(45) Veröffentlichungstag und Bekanntmachung des Hinweises auf die Patenterteilung: **02.06.2021**
(21) Anmeldenummer: 17742162.5
(22) Anmeldetag: 18.07.2017
(51) Int. Cl.: A61M 1/16, A61M 1/36

(54) **DIALYSEGERÄT MIT EINER STEUEREINHEIT ZUR DURCHFÜHRUNG EINER KONDITIONIERUNG DER DIALYSEMEMBRAN**
DIALYSIS DEVICE HAVING A CONTROL UNIT FOR PERFORMING CONDITIONING OF THE DIALYSIS MEMBRANE
APPAREIL DE DIALYSE COMPRENANT UNE UNITÉ DE COMMANDE POUR RÉALISER UN CONDITIONNEMENT DE LA MEMBRANE DE DIALYSE

(30) Priorität: 18.07.2016 DE 102016008755
(43) Veröffentlichungstag der Anmeldung: 22.05.2019
(73) Patentinhaber: Fresenius Medical Care Deutschland GmbH, 61352 Bad Homburg (DE)
(72) Erfinder: SPICKERMANN, Reiner, 97535 Wasserlosen-Burghausen (DE); GAGEL, Alfred, 96123 Litzendorf (DE)
(74) Vertreter: Herrmann, Uwe
(86) Internationale Anmeldenummer: PCT/EP2017/000868
(87) Internationale Veröffentlichungsnummer: WO 2018/015010

(56) Entgegenhaltungen:
- EP-A1- 1 457 218
- EP-A1- 2 583 700
- US-A- 5 932 103
- US-A1- 2009 101 576

## Beschreibung

Die Erfindung betrifft ein Dialysegerät mit einem extrakorporalen Blutkreislauf, einem Dialysator und einem Dialysatkreislauf, wobei im extrakorporalen Blutkreislauf eine Blutpumpe angeordnet ist.

Dialysegeräte sind aus dem Stand der Technik, beispielsweise aus der Druckschrift US5932103A bekannt. Auch die EP1457218 offenbart ein Dialysegerät sowie ein zugehöriges Füllverfahren. Die Druckschrift EP2583700A1 bezieht sich auf ein Verfahren zum Starten einer Hämodialyse unter Verwendung eines Hämodialysegerätes. Die US2009/101576 A1 offenbart zudem ein Primingverfahren für ein Dialysegerät.

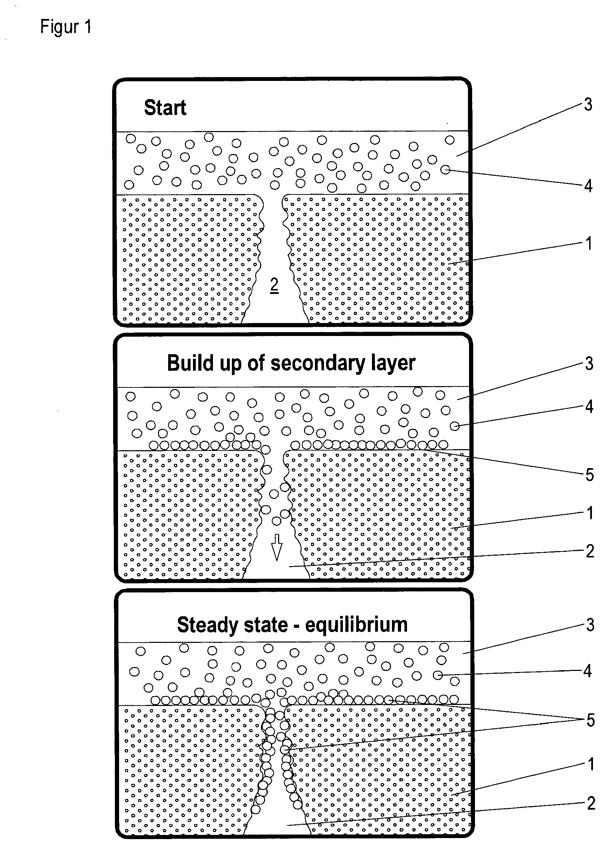

Zu Beginn einer Dialysebehandlung, beispielsweise Hämodialyse- oder Hämodiafiltrationsbehandlung zeigen Dialysatoren eine sehr hohe Durchlässigkeit für größere Moleküle, unter anderem für Albumin. Durch den Kontakt mit bestimmten Blutbestandteilen, insbesondere Blutproteinen wird die Dialysemembran verändert, so dass die Siebwirkung für große Proteine wie Albumin stark verbessert wird. Im Detail bildet sich durch die Ablagerung von Albumin und anderen Blutbestandteilen auf der Dialysemembran eine Sekundärmembran, die zu einer erwünschtermaßen besseren Siebwirkung für große Proteine wie Albumin führt. Dieser Veränderungsvorgang ist schematisch in Figur 1 dargestellt, wobei die obere Abbildung die Membran 1 mit Membranpore 2 beim Start der Behandlung zeigt. In einer frühen Phase der Behandlung bauen die im Blut 3 befindlichen Plasmaproteine 4 dann eine Sekundärschicht 5 um die Pore 2 und in der Pore 3 auf (mittlere Abbildung), bis ein Steady State erreicht wird (untere Abbildung).

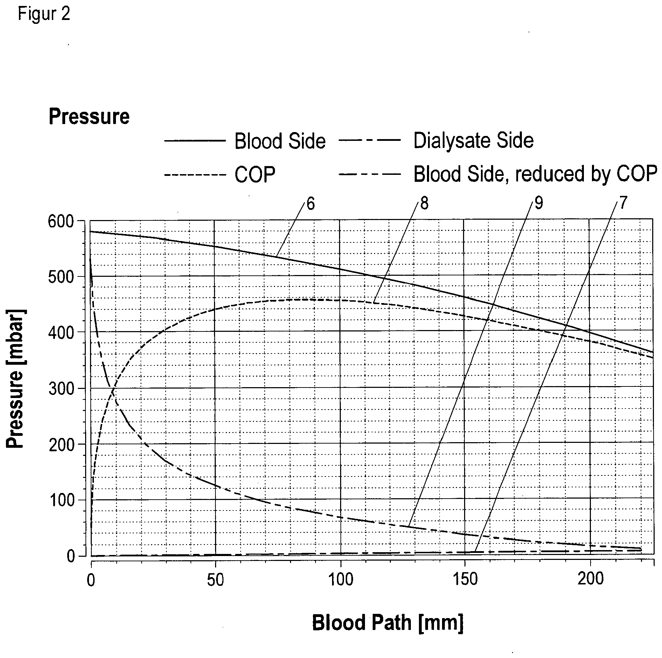

Die Ausprägung der oben beschriebenen Membrankonditionierung bei beispielsweise HDF-Therapien wird von der Höhe und der Dauer der Substitutionsrate bestimmt. Je höher die Austauschrate ist, umso besser und schneller verläuft die Konditionierung der Membran. Allerdings unterscheiden sich die lokalen Filtrationsleistungen längs der Dialysatormembran sehr, weil der lokale Transmembrandruck (TMP) und der lokale onkotische Druck variieren. Dies lässt sich in Figur 2 erkennen, die Verläufe der hydrostatischen Drücke auf der Blutseite (p_{B}, Bezugszeichen 6) und auf der Dialysatseite (p_{D}, Bezugszeichen 7), des onkotischen Drucks (COP; Bezugszeichen 8) und des effektiven Drucks (p_{B}-COP; Bezugszeichen 9) längs der Dialysatormembran zeigt.

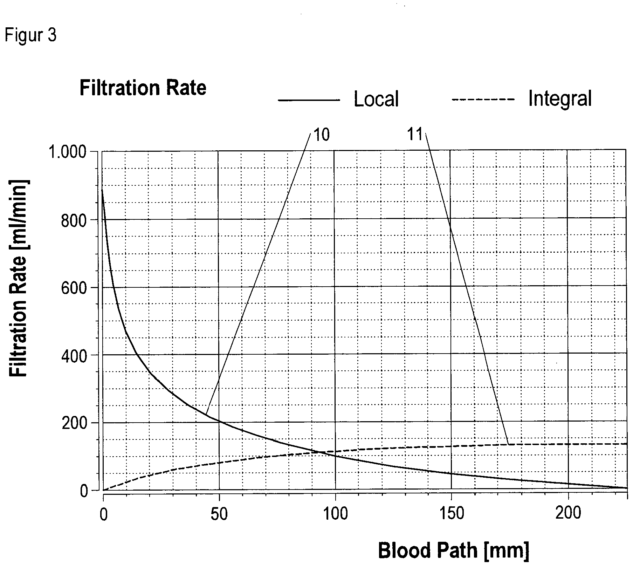

Während die lokale Filtration am blutseitigen Eingang des Dialysators sehr hoch ist, fällt sie längs des Dialysators bzw. der Kapillaren schnell ab und strebt am Ausgang des Dialysators gegen Null oder kann sogar negativ werden (in diesem Fall spricht man von einer Backfiltration). Die Verläufe der lokalen Filtration am blutseitigen Eingang (Bezugszeichen 10) und der integralen Filtration über die zurückgelegte Strecke an der Dialysatormembran (Bezugszeichen 11) sind in Figur 3 dargestellt. Entsprechend starke Unterschiede sind in beispielsweise der Geschwindigkeit und Qualität der Konditionierung des Filters gemäß Figur 1 zu erwarten.

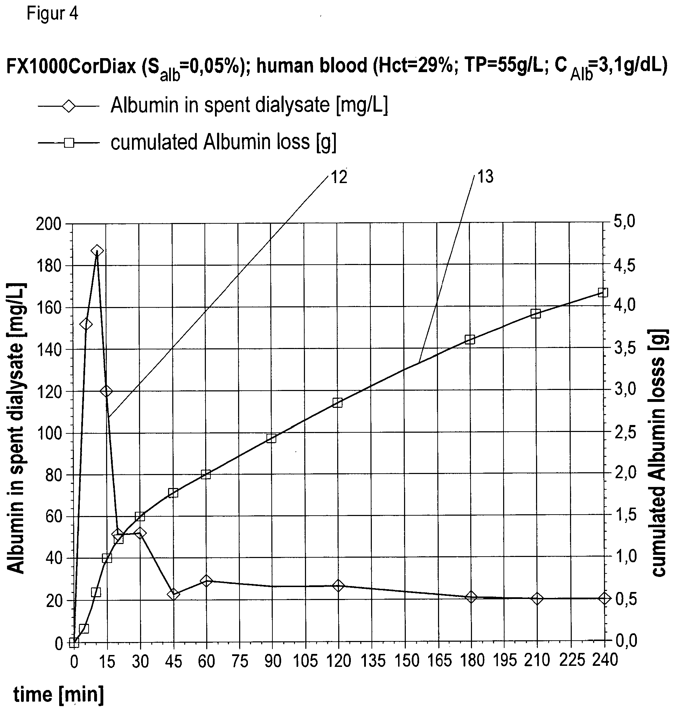

Der (ausgangsnahe) Bereich des Filters, der unter Umständen nur mäßig konditioniert worden ist, kann während der Behandlungszeit einen relativ hohen Beitrag zum Albuminverlust verursachen, auch wenn das Austauschvolumen in diesem Bereich gering ist. Mit Albuminverlust ist vorliegend insgesamt der Verlust an Plasmaproteinen gemeint, von denen Albumin aber der größte und wichtigste Anteil ist. Daher wird auf diesen vorliegend stellvertretend Bezug genommen. Dies ist in Figur 4 zu erkennen, wo gemessene zeitliche Verläufe der momentanen Albuminkonzentration (Bezugszeichen 12) und der kumulierten Albuminkonzentration (Bezugszeichen 13) im Dialysat dargestellt sind.

Aufgabe der Erfindung ist, ein Dialysegerät bereitzustellen, mit dem erreicht wird, dass die lokalen Siebkoeffizienten an allen Stellen der Membran gleichmäßiger reduziert werden.

Vor diesem Hintergrund betrifft die Erfindung ein Dialysegerät mit einem extrakorporalen Blutkreislauf, einem Dialysator und einem Dialysatkreislauf, wobei der Dialysator eine Dialysemembran aufweist, welche seine Blutseite von seiner Dialysatseite trennt. Erfindungsgemäß ist vorgesehen, dass das Dialysegerät eine Steuereinheit aufweist, die ausgebildet ist, einen Konditionierungszyklus durchzuführen, der eine Konditionierungsphase umfasst, in der eine Ultrafiltrationsrate den Blutfluss, der durch die Blutpumpe gefördert wird, durch den Dialysator übersteigt. Es wird also ein Druckgradient über die Dialysemembran erzeugt, der mit einem Flüssigkeitsentzug des Blutes einhergeht. Die Ultrafiltrationsrate kann beispielsweise anhand einer Ultrafiltrationspumpe geregelt werden. Alternativ oder zusätzlich können auch andere Aktoren wie beispielsweise Substitutionspumpen in die Regelung eingreifen. Die Ultrafiltrationsrate wird vorliegend allgemein als die Rate des Flüssigkeitsentzugs verstanden.

Das Dialysegerät weist eine im extrakorporalen Blutkreislauf angeordnete Blutpumpe auf, die den Blutfluss regelt. Diese kann sich im arteriellen Zweig des extrakorporalen Blutkreislaufs befinden.

Die Ultrafiltrationsrate während der Konditionierungsphase kann einer normalen, d.h., einer auch im Rahmen der Behandlung bestehenden Ultrafiltrationsrate entsprechen oder davon abweichen. Sie kann beispielsweise zwischen 30 ml/min und 150 ml/min und vorzugsweise zwischen 50 ml/min und 100 ml/min betragen. Beispielhafte Werte umfassen 60 ml/min und 90 ml/min.

Durch die geringe Flussrate sind die Druckunterschiede über die Länge des Dialysators nur mehr gering, sodass eine über die Länge des Dialysators annähernd gleichmäßige oder zumindest gleichmäßigere Beaufschlagung der Membran mit Blutproteinen und insbesondere Albumin erfolgt. Somit erfolgt die eingangs beschriebene Membrankonditionierung gleichmäßiger bzw. die Sekundärmembran bildet sich gleichmäßig aus. Wenn dieser Vorgang mit Beginn einer Dialyse stattfindet, kann der Albuminverlust insbesondere zu Beginn der Behandlung, in dem die Membran normalerweise nicht oder nur unregelmäßig konditioniert ist, signifikant verringert werden.

Bei dem Dialysator kann es sich um einen Kapillardialysator handeln.

In einer Ausführungsform wird der Konditionierungszyklus mit Beginn der Behandlung durchgeführt. Zu Beginn der Behandlung und bei Durchführung des Konditionierungszyklus ist der Blutkreislauf schon vollständig mit Blut gefüllt bzw. das Dialysegerät schon am Patienten angeschlossen. Ferner ist auch der Dialysatkreislauf zu Beginn des Konditionierungszyklus mit Flüssigkeit gefüllt.

Bei der (Dialyse-)Behandlung kann es sich beispielsweise um eine Hämodialysebehandlung oder um eine Hämodiafiltrationsbehandlung handeln.

In einer Ausführungsform übersteigt die Ultrafiltrationsrate während der Konditionierungsphase den Blutfluss durch den Dialysator um ein bestimmtes Maß. Beispielsweise kann das Verhältnis der Ultrafiltrationsrate im Dialysator zur Blutflussrate zwischen 1,5 zu 1 und 2,5 zu 1, vorzugsweise zwischen 1,8 zu 1 und 2,2 zu 1 und vorzugsweise etwa 2 zu 1 sein. Dann erfolgt eine Eindickung des Bluts im Dialysator und ein Rückfluss von Blut aus der venösen Leitung. Die Druckunterschiede im Dialysator sind gering, was zu einer gleichmäßigen Filtration über die Länge des Dialysators führt.

In einer Ausführungsform ist vorgesehen, dass während der Konditionierungsphase wenig oder kein Dialysat in die Dialysatseite des Dialysators fließt. Beispielsweise kann die Zuflussrate weniger als 1000 ml/min oder weniger als 300 ml/min betragen. Der Zufluss entspricht dem Standardverfahren der Hämofiltration. Zum Erzielen einer gleichmäßigeren Schicht in der Konditionierungsphase ist auch auf der Dialysatseite ein möglichst kleiner Druckgradient über die Länge des Dialysators wünschenswert, damit die lokalen Ultrafiltrationsraten längs des Dialysators möglichst gleich groß sind. Dies wird in dieser Ausführungsform bei geringem bzw. ausgeschaltetem Dialysatfluss erreicht. Am Dialysatorausgang wird dann nur oder im Wesentlichen nur das Ultrafiltrat abgezogen.

In einer Ausführungsform beträgt die Dauer der Konditionierungsphase 15 bis 120 Sekunden und vorzugsweise 30 bis 60 Sekunden.

In einer Ausführungsform wird während der Konditionierungsphase ein Flüssigkeitsvolumen von zwischen 20 % und 70 % und vorzugsweise von zwischen 30 % und 60 % des Gesamtvolumens der Blutseite des Dialysators durch die Dialysemembran abgezogen. Hierdurch erfolgt eine temporäre Eindickung des Bluts im Dialysator. Beispielsweise kann vorgesehen sein, dass 1/2 oder 1/3 des Gesamtvolumens auf der Blutseite des Dialysators durch die Dialysemembran abgezogen wird.

Das Gesamtvolumen auf der Blutseite des Dialysators kann zwischen 50 und 200 ml und vorzugsweise zwischen 80 und 150 ml liegen. Beispielhafte Werte umfassen 95 ml, 115 ml oder 136 ml. Diese Werte hängen mit der Größe des Filters zusammen. Das Flüssigkeitsvolumen, das während der Konditionierungsphase abgezogen wird, kann zwischen 15 und 80 ml und vorzugsweise zwischen 25 und 70 ml liegen. Diese Werte hängen von den Flussraten der Pumpen und dem sich daraus ableitenden Transmembrandruck ab.

Der Transmembrandruck kann auch dazu genutzt werden, um Substitutionsraten und indirekt die Blutflussrate zu regeln. Bei Überschreitung eines Grenzwertes für den Transmembrandruck können Vorkehrungen zur Reduktion getroffen werden oder eine Abbruch stattfinden.

In einer Ausführungsform wird der Konditionierungszyklus wiederholt, vorzugsweise mindestens 3x und beispielsweise zwischen 3x und 6x. Vorzugsweise ist vorgesehen, dass die Wiederholungen unmittelbar aufeinander folgen. Dadurch kann die Konditionierung der Membran weiter verbessert werden. Sofern mehrere vorzugsweise aufeinander folgende Konditionierungszyklen vorgesehen sind, können diese alle mit Behandlungsbeginn stattfinden.

In einer Ausführungsform umfasst der Konditionierungszyklus eine Spülphase, die auf die Konditionierungsphase folgt und in welcher der Blutfluss durch den Dialysator die Ultrafiltrationsrate übersteigt. Beispielsweise kann vorgesehen sein, dass die Ultrafiltrationspumpe in dieser Phase ausgeschaltet ist oder mit gegenüber der Konditionierungsphase verringerter oder gleicher Rate betrieben wird. In dieser Phase wird das eingedickte Blut aus dem Dialysator geschwemmt. Vorzugsweise startet die Spülphase unmittelbar nach der Konditionierungsphase. So kann durch frisches Blut ein weiterer Schichtaufbau erfolgen und eine ständige Eindickung, die zu Clotting führen würde, wird vermieden.

In einer Ausführungsform kann das Blutvolumen, das in der Spülphase verwendet wird, um die Blutkammer des Dialysators zu spülen, das 0,5 bis 5-fache und vorzugsweise das 1 bis 2-fache des Blutkammervolumens betragen.

In einer Ausführungsform umfasst der Konditionierungszyklus nur die Konditionierungsphase und die Spülphase. Während der Phasen oder zwischen den Phasen können Messungen vorgenommen werden.

Das erfindungsgemäße Dialysegerät weist eine Substitutionsleitung auf, die vorzugsweise in die venöse Leitung des extrakorporalen Blutkreislaufs führt, wobei die Steuereinheit derart ausgebildet ist, dass anhand dieser Leitung das in der Konditionierungsphase im Dialysator abgezogene Flüssigkeitsvolumen wenigstens teilweise substituiert wird, vorzugsweise in der Konditionierungsphase und/oder während der Spülphase.

Diese Offenbarung betrifft ferner ein nicht-erfindungsgemäßes Verfahren zur Durchführung einer Dialysebehandlung an einem erfindungsgemäßen Gerät, wobei ein Konditionierungszyklus durchgeführt wird, wie er im Zusammenhang mit dem erfindungsgemäßen Gerät beschrieben wurde.

Es lässt sich festhalten, dass anhand der vorliegenden Erfindung ein gleichmäßiger Aufbau einer Sekundärmembran über die Membranoberfläche des Dialysators erreicht werden kann. Ferner kann die Sekundärmembran schneller gebildet werden.

Die Albuminverluste können initial und kumulativ in einer nachfolgenden Dialysebehandlung minimiert werden.

Ferner kann die Hämokompatibilität erhöht werden. Denn wenn Blut mit Fremdoberflächen in Kontakt tritt, kann es zu vielfältigen Reaktionen kommen. Wird eine Sekundärmembran aber wie in der vorliegenden Erfindung schnell und gleichmäßig aufgebaut, so kommt das Blut im Dialysator an größeren Flächenbereichen nur noch mit bluteigenen Stoffen in Kontakt.

Zusätzlich wird durch die Verkleinerung des Siebkoeffizienten der Verlust von Stoffen, die nicht entfernt werden sollen, beispielsweise von Enzymen oder Hormonen, reduziert.

Weitere Einzelheiten und Vorteile der Erfindung ergeben sich aus den nachfolgend diskutierten Figuren und Ausführungsbeispielen. In den Figuren zeigen:
- Figur 1:: eine schematische Darstellung des Veränderungsvorgangs, wenn die Dialysemembran in einer frühen Behandlungsphase in Kontakt mit Blutproteinen kommt;
- Figur 2:: Verläufe der hydrostatischen Drücke auf der Blut- und Dialysatseite, des onkotischen Drucks und des effektiven Drucks längs der Dialysatormembran;
- Figur 3:: Verläufe der lokalen Filtration am blutseitigen Eingang und der integralen Filtration über die zurückgelegte Strecke an der Dialysatormembran;
- Figur 4:: zeitliche Verläufe der momentanen Albuminkonzentration und der kumulierten Albuminkonzentration im Dialysat;
- Figur 5:: einen Versuchsaufbau für die experimentelle Austestung der Erfindung;
- Figur 6: eine schematische Darstellung der Fluss- und Druckverläufe im Dialysator bei einem Verhältnis der Ultrafiltrationsrate zur Blutflussrate von 2 zu 1;
- Figur 7:: eine graphische Darstellung der zeitlichen Verläufe ermittelter Albuminkonzentrationen im Ultrafiltrat; und
- Figur 8:: eine schematische Darstellung eines erfindungsgemäßen Dialysegeräts.

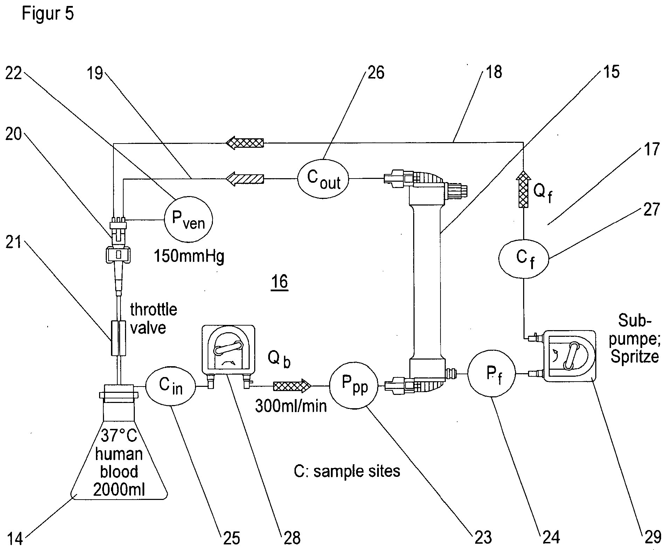

In Figur 5 wird ein Versuchsaufbau für die experimentelle Austestung der Erfindung. Mit dem Bezugszeichen 14 ist ein Gefäß gekennzeichnet, in dem Blut mit Körpertemperatur von etwa 37°C in einer Menge von beispielsweise 2000 ml befinden kann. Mit dem Bezugszeichen 15 ist ein Dialysator gekennzeichnet, mit dem Bezugszeichen 16 ein simulierter extrakorporaler Blutkreislauf und mit dem Bezugszeichen 17 ein simulierter Dialysatkreislauf. In der Versuchsanordnung schließt der Dialysatkreislauf 17 mit der Abflussleitung 18 zur Substitution des Dialysats an die venöse Leitung 19 des Blutkreislaufs 16 an, und zwar an einer Tropfkammer 20. Zwischen Tropfkammer 20 und dem Gefäß 14 befindet sich noch ein Drosselventil 21.

Drucksensoren 22, 23 und 24 dienen der Ermittlung der Drücke Pᵥₑₙ, Ppp und P_{f}. Die Bezugszeichen 25, 26 und 27 bezeichnen Messpunkte für die Konzentrationen Cᵢₙ, Cₒᵤₜ und C_{f}. Anhand der stromaufwärts des Dialysators 15 im Blutkreislauf 16 angeordneten Blutpumpe 28 kann ein Blutfluss Q_{b} von beispielsweise 300 ml/min erzeugt werden. Anhand der stromabwärts des Dialysators 15 im Dialysatkreislauf 17 angeordneten Ultrafiltrationspumpe 29, bei der es sich im Versuchsaufbau gleichzeitig um eine Substitutionspumpe bzw. Spritze handelt, kann ein Dialysat- bzw. Substituatfluss Qf erzeugt werden.

Das venöse Schlauchsystem 19 muss luftfrei mit dem ("Patienten"-)Volumen verbunden sein, so dass über den venösen Zweig ein Volumenstrom von beispielsweise 40 ml/min zurückgezogen werden kann. Der Druck Pᵥₑₙ kann beispielsweise 150 mmHg betragen.

Mit dieser Anordnung wurden die nachfolgenden Versuche durchgeführt. Bei allen Versuchen waren die folgenden Parameter annähernd identisch:
Dialysatortyp: FX600 (V_{b} = 95 mL)
Siebkoeffizient: S_{alb} = 0,032 %
Blutfluss: Q_{b} = 300 ml/min
Hämatokrit: Hkt = 36 % ± 2 %
Albumin: C_{alb} ≥ 3,0 g/dL

### Beispiel 1:

Zunächst wurde der Versuchsaufbau gemäß Figur 5 herstellt (Schritt 1). Der Dialysator 15 wurde blut- und dialysatseitig mit NaCI-Lösung gefüllt und gespült und Restluft wurde auf beiden Seiten des Dialysators 15 vollständig durch Klopfen entfernt (Schritt 2). Anschließend wurde die Blutseite mit Blut gefüllt (Schritt 3).

In Übereinstimmung mit der Erfindung wurde sodann die Blutpumpe 28 gestoppt (Schritt 4), um Druckabfälle längs der Kapillaren des Dialysators 15 abzubauen.

Mit einer Substitutionspumpe 29 wurden etwa 1/3 des Volumens auf der Blutseite über die Membran abgezogen (Schritt 5). Der Wert für ΔV_{uf} betrug 30 ml. Das Ultrafiltrationsvolumen ΔV_{uf} wird somit gleichmäßig über die Membran gezogen. Der Ablauf war wie folgt: Die Substitutionspumpe 29 zieht mit Q_{uf} = 60 ml/min auf der Dialysatseite, bis das gewünschte Ultrafiltrations-(UF-)Volumen von 30 ml pro Zyklus abgezogen worden ist (Dauer ca. 30 s bis 60 s). Die Blutpumpe 28 läuft in dieser Phase mit der halben UF-Rate mit Q_{b} = 30 ml/min. Über das venöse Schlauchsystem 19 wird der restliche Volumenstrom (ca. 30 ml/min) zurückgezogen .

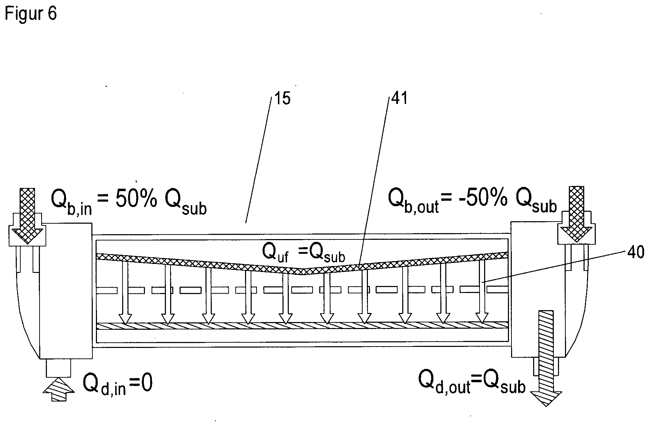

Das Verhältnis der UF-Rate Q_{uf} (60 ml/min) zur Vorlaufflussrate des Blutes Q_{b,in} (30 ml/min) beträgt in diesem Beispiel genau 2/1. Figur 6 zeigt schematisch den Flussverlauf 40 und Druckverlauf 41 im Dialysator 15, der sich bei einem derartigen Verhältnis ergibt. Der Fluss Q_{d,out} des aus dem Blut abgezogenen Filtrats, der bei einer Dialysatzuflussrate Q_{d,in} von 0 ml/min genau der Ultrafiltrationsrate Q_{uf} entspricht, stammt zur Hälfte aus der arteriellen Leitung, wo die Blutpumpe 28 Blut nachliefert. Da die Blutkammer des Dialysators 15 offen mit der venösen Leitung 19 verbunden ist, wird auch aus der venösen Leitung 19 Blut angesaugt (30 ml/min). Durch die symmetrische Verteilung werden möglichst gleichmäßige Druckverhältnisse für eine möglichst gleichmäßige Schicht erzielt. Der Druckgradient auf der Blutseite des Dialysators ist so am geringsten.

Im Anschluss erfolgt eine Bestimmung des Albuminverlusts im Ultrafiltrationsvolumen anhand der Formel Δm_{alb} = C_{alb} * ΔV_{uf} (Schritt 6). Das entzogene Proben-Ultrafiltrationsvolumen ΔV_{uf} wieder ins Vorratsgefäß 14 zurückgeben.

Sodann wird die Blutpumpe 28 wieder mit Q_{b} = 300 ml/min gestartet (Schritt 7). Damit wird das eingedickte Blut wieder aus dem Dialysator 15 ausgespült.

Nach 2 min (600 ml Blutvolumen) wird die Blutpumpe 28 erneut gestoppt (Schritt 8).

Nach Schritt 8 kann entweder der Konditionierungs-Zyklus der Schritte 5 bis 8 wiederholt werden oder ein Übergang zu Schritt 9 erfolgen. Im vorliegenden Ausführungsbeispiel wird der Konditionierungs-Zyklus der Schritte 5 bis 8 insgesamt 5-mal durchlaufen, also beträgt das gesamte V_{uf} im Konditionierungszyklus 150 ml und es werden insgesamt 5 Proben gezogen.

Im abschließenden Schritt 9 wird eine Post-HF-Behandlung mit einer Substitutionsrate von Q_{sub} = 100 ml/min und einer Dauer von T = 180 min simuliert. Dabei werden zu Testzeitpunkten (t = 0 min; t = 2 min 30 sek; t = 5 min; t = 7 min 30 sek; t = 10 min; t = 15 min; t = 20 min; t = 30 min; t = 45 min; t = 60 min; t = 90 min; t = 120 min; t = 150 min; t = 180 min) Proben aus dem Ultrafiltrat (C_{f}, Messpunkt 25) und dem Vorratsgefäß (Cᵢₙ; Messpunkt 27) gezogen und die ungestörten Drücke Pᵥₑₙ, P_{PP} und P_{f} notiert.

Die Auswertung erfasste (a) den Albuminverlust und Myoglobinverlust während der Konditionierungsphase, d.h., während der 5-fachen Wiederholung der Schritte 5 bis 8, und (b) den Albuminverlust während der 3-stündigen Behandlung.

### Beispiel 2:

Dieses Beispiel war in weiten Teilen identisch dem Beispiel 1, der Konditionierungs-Zyklus, d.h., Schritte 5 bis 8, wurden aber insgesamt 15-mal wiederholt, also beträgt das gesamte V_{uf} im Konditionierungszyklus 450 mL und der Ablauf des Schrittes 5 war wie folgt: Die Substitutionspumpe 29 zieht mit Q_{uf} = 90 ml/min auf der Dialysatseite, bis das gewünschte Ultrafiltrations-(UF-)Volumen von 30 ml pro Zyklus abgezogen worden ist (Dauer wiederum ca. 30 s bis 60 s). Die Blutpumpe 28 läuft in dieser Phase mit der halben UF-Rate mit Q_{b} = 45 ml/min. Über das venöse Schlauchsystem 19 wird der restliche Volumenstrom (ca. 45 ml/min) zurückgezogen.

Die Auswertung erfolgte wie in Beispiel 1.

Gemäß einem abgewandelten Beispiel 2' wären auch eine Einstellung von Q_{b} bei 40 ml/min denkbar, was bei einer Ultrafiltrationsrate Q_{uf} = 90 ml/min zu einer Rückflussrate aus der venösen Leitung von 50 ml/min führen würde.

### Beispiel 3:

Auch dieses Beispiel war in weiten Teilen identisch dem Beispiel 1, aber mit der Spritze bzw. Substitutionspumpe 29 wurden hier in Schritt 5 etwa 1/2 des Volumens auf der Blutseite über die Membran abgezogen, also ΔV_{uf} = 45 ml, bei 5-maligem Durchlaufen des Konditionierungs-Zyklus der Schritte 5 bis 8 also 225 m.

Die Auswertung erfolgte wiederum wie in Beispiel 1.

### Vergleichsbeispiel 4:

Nach Durchführung der Schritte 1 bis 3 gemäß Beispiel 1 wurden die Schritte 4 bis 8 ausgelassen und eine Post-HF-Behandlung mit einer Substitutionsrate von Q_{sub} = 100 ml/min wurde wie in Schritt 9 simuliert.

Die Auswertung erfolgte wiederum wie in Beispiel 1.

Dieselbe Versuche wie in Beispielen 1 bis 4 könnten auch mit alternativen Dialysatoren durchgeführt werden, beispielsweise mit einem FX800 (V_{b} = 115 ml) oder einem FX1000 (V_{b} = 136 ml). In Beispiel 1, Schritt 5 würde das Ultrafiltrationsvolumen ΔV_{uf} dann 37 ml (FX800) bzw. 44 ml (FX1000) betragen und bei 5-maliger Wiederholung des Konditionierungszyklus käme man auf ein gesamtes ΔV_{uf} von 185 ml (FX800) bzw. 220 ml (FX1000). In Beispiel 2, also bei 15-maliger Wiederholung des Konditionierungszyklus käme man auf ein gesamtes ΔV_{uf} von 555 ml (FX800) bzw. 660 ml (FX1000). In Beispiel 3 würde das Ultrafiltrationsvolumen ΔV_{uf} dann 55 ml (FX800) bzw. 65 ml (FX1000) betragen und bei 5-maliger Wiederholung des Konditionierungszyklus käme man auf ein gesamtes ΔV_{uf} von 275 ml (FX800) bzw. 325 ml (FX1000).

Die für Beispiele 1 bis 4 gemessenen absoluten und relativen Albumin- und Myoglobinverluste über die gesamte Behandlungsdauer (bestimmt im Ultrafiltrat an Messpunkt 27) sind in Tabelle 1 dargestellt.

**Tabelle 1:**

| Beispiel | Albuminverlust absolut | Albuminverlust relativ | Myoglobinverlust absolut | Myoglobinverlust relativ |
|---|---|---|---|---|
| V4 | 1,08 g | | 22,3 mg | |
| V4 (Doppel) | 1,24 g | | 20,7 mg | |
| 1 | 0,85 g | - 26 % | 21,2 mg | - 1 % |
| 2 | 0,77 g | - 33 % | 21,2 mg | -1 % |
| 3 | 0,68 g | - 41 % | 20,3 mg | -6 % |

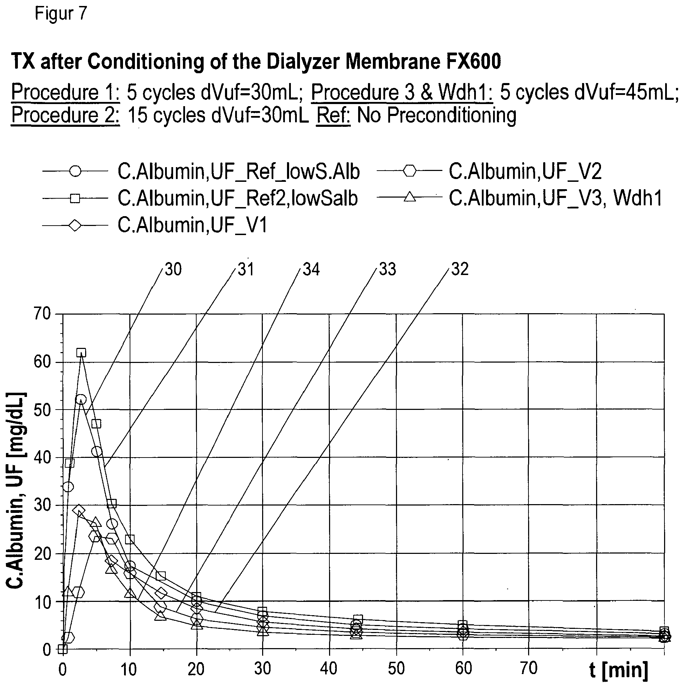

Die zeitlichen Verläufe der an Messpunkt 27 ermittelten Albuminkonzentrationen im Ultrafiltrat sind in Figur 7 dargestellt, wobei das Bezugszeichen 30 die Kurve für Vergleichsbeispiel 4, Bezugszeichen 31 die Kurve für das Doppel des Vergleichsbeispiels 4, Bezugszeichen 32 die Kurve für Beispiel 1, Bezugszeichen 33 die Kurve für Beispiel 2 und Bezugszeichen 34 die Kurve für Beispiel 3 zeigen.

Die Mittel der über die Behandlungszeit gemessenen Transmembrandrücke TMP und Differenzen P_{PP}-Pᵥₑₙ sind in Tabelle 2 dargestellt.

**Tabelle 2:**

| Beispiel | TMP | P_{PP}-Pᵥₑₙ |
|---|---|---|
| V4 | | |
| V4 (Doppel) | 190 mmHg | 161 mmHg |
| 1 | 150 mmHg | 150 mmHg |
| 2 | 174 mmHg | 161 mmHg |
| 3 | 160 mmHg | 158 mmHg |

Zusammenfassend lässt sich anhand der Beispiele festhalten, dass die Albuminverluste während des Konditionierungs-Zyklus wegen der geringen ausgetauschten Volumina vernachlässigbar waren (ΔV_{uf} = 5*30 ml = 150 ml; C_{alb,max} = 0,5 mg/ml; Δm_{alb} = 75 mg). Die beste Konditionierung lieferte Beispiel 3 mit einem Wert für ΔV_{uf} von 5*45 ml (50% des Blutvolumens) und einer Reduktion des Albuminverlusts um 41 %. Der Konditionierungszyklus dauert pro Wiederholung inklusive Spülen ca. 2 bis 3 min, bei 5-maliger Wiederholung also 10 bis 15 min.

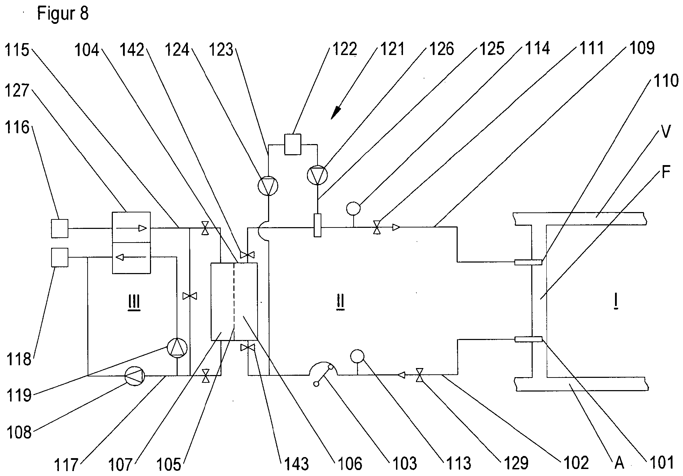

Figur 8 zeigt eine schematische Darstellung eines erfindungsgemäßen Dialysegeräts.

In der Figur sind mit dem Bezugszeichen I das Blutsystem des Patienten, mit dem Bezugszeichen II der extrakorporale Blutkreislauf des Geräts und mit Bezugszeichen III die Dialysatseite des Geräts bezeichnet.

An einem derartigen Gerät kann das anhand des oben näher beschriebenen Versuchsaufbaus erprobte Prinzip im Sinne der Erfindung umgesetzt werden.

Das Blutsystem I des Patienten umfasst eine Arterie A, eine Vene V und eine Fistel F.

Der extrakorporale Blutkreislauf ist anhand der Nadeln 101 und 110 mit der Fistel verbunden. An die arterielle Nadel 101 schließt die arterielle Leitung 102 an, die eine Blutpumpe 103 aufweist und stromabwärts in den Dialysator 104 mündet. Aus dem Dialysator 104 läuft die venöse Leitung 109 zur venösen Nadel 110. Der extrakorporale Blutkreislauf umfasst arterielle und venöse Drucksensoren 113 und 114 sowie Klemmen 129, 143, 142 und 111.

Der Dialysator 104 umfasst eine Dialysatormembran 105, durch welche die Blutkammer 106 von der Dialysatkammer 107 getrennt ist.

Das Dialysatsystem III umfasst eine Dialysatquelle 116, eine Bilanziereinrichtung 127 und eine Zuleitung 115, die in die Dialysatkammer 107 des Dialysators 104 führt. In eine Ableitung 117 aus der Dialysatkammer 107 sind die Ultrafiltrations- bzw. Dialyseflüssigkeitspumpe 108 und 119 angeordnet. Der Rücklauf ist mit dem Bezugszeichen 118 gekennzeichnet.

Ein Substitutionsflüssigkeitssystem 121 umfasst eine Substitutionsflüssigkeitsquelle 122 sowie eine Prädilutionsleitung und -pumpe 123 bzw. 124 und eine Postdilutionsleitung und -pumpe 125 bzw. 126.

Das gezeigte Dialysegerät stellt im Gegensatz zu Figur 5 nicht bloß einen Versuchsaufbau dar, sondern ein reales erfindungsgemäßes Gerät, an dem das erfinderische Prinzip zur Anwendung kommen kann.

## Patentansprüche

1. Dialysegerät mit einem extrakorporalen Blutkreislauf, einem Dialysator (15) und einem Dialysatkreislauf (17), wobei im extrakorporalen Blutkreislauf (16) eine Blutpumpe (18) angeordnet ist, wobei der Dialysator (15) eine Dialysemembran aufweist, welche seine Blutseite von seiner Dialysatseite trennt,
**dadurch gekennzeichnet,**
**dass** das Dialysegerät eine Steuereinheit aufweist, die ausgebildet ist, einen Konditionierungszyklus durchzuführen, der eine Konditionierungsphase umfasst, in der eine Ultrafiltrationsrate den durch die Blutpumpe (28) geförderten Blutfluss durch den Dialysator (15) übersteigt, und dass das Dialysegerät eine Substitutionsleitung aufweist, die vorzugsweise in die venöse Leitung des extrakorporalen Blutkreislaufs (16) führt, wobei die Steuereinheit derart ausgebildet ist, dass anhand dieser Leitung das in der Konditionierungsphase im Dialysator (15) abgezogene Flüssigkeitsvolumen wenigstens teilweise substituiert wird, vorzugsweise nach der Konditionierungsphase und insbesondere während einer Spülphase.

2. Dialysegerät nach Anspruch 1, **dadurch gekennzeichnet, dass** der Konditionierungszyklus mit Beginn der Behandlung durchgeführt wird.

3. Dialysegerät nach einem der vorhergehenden Ansprüche, **dadurch gekennzeichnet, dass** das Verhältnis der Ultrafiltrationsrate zur durch die Blutpumpe bedingten Blutflussrate zwischen 1,5 zu 1 und 2,5 zu 1, vorzugsweise zwischen 1,8 zu 1 und 2,2 zu 1 und vorzugsweise etwa 2 zu 1 beträgt.

4. Dialysegerät nach einem der vorhergehenden Ansprüche, **dadurch gekennzeichnet, dass** während der Konditionierungsphase wenig oder kein Dialysat in die Dialysatseite des Dialysators (15) fließt.

5. Dialysegerät nach einem der vorhergehenden Ansprüche, **dadurch gekennzeichnet, dass** die Dauer der Konditionierungsphase 15 bis 120 Sekunden und vorzugsweise 30 bis 60 Sekunden beträgt.

6. Dialysegerät nach einem der vorhergehenden Ansprüche, **dadurch gekennzeichnet, dass** während der Konditionierungsphase ein Flüssigkeitsvolumen von zwischen 20 % und 70 % und vorzugsweise von zwischen 30 % und 60 % des Gesamtvolumens der Blutseite des Dialysators (15) durch die Dialysemembran abgezogen wird.

7. Dialysegerät nach einem der vorhergehenden Ansprüche, **dadurch gekennzeichnet, dass** der Konditionierungszyklus wiederholt wird, vorzugsweise mindestens 3x und beispielsweise zwischen 3x und 6x, wobei die Wiederholungen vorzugsweise unmittelbar aufeinander folgen.

8. Dialysegerät nach einem der vorhergehenden Ansprüche, **dadurch gekennzeichnet, dass** der Konditionierungszyklus eine Spülphase umfasst, die auf die Konditionierungsphase folgt und in welcher der durch die Blutpumpe (18) erzeugte Blutfluss durch den Dialysator (15) die Ultrafiltrationsrate übersteigt.

9. Dialysegerät nach Anspruch 8, **dadurch gekennzeichnet, dass** das Blutvolumen, das in der Spülphase verwendet wird, um die Blutkammer des Dialysators (15) zu spülen, das 0,5 bis 5-fache und vorzugsweise das 1 bis 2-fache des Blutkammervolumens beträgt.

## Claims

1. A dialysis machine having an extracorporeal blood circuit, a dialyzer (15), and a dialysate circuit (17), wherein a blood pump (18) is arranged in the extracorporeal blood circuit (16); and wherein the dialyzer (15) has a dialysis membrane which separates its blood side from its dialysate side,
**characterized in that**
the dialysis machine has a control unit which is configured to carry out a conditioning cycle which comprises a conditioning phase in which an ultrafiltration rate exceeds the blood flow through the dialyzer (15) which is conveyed by the blood pump; and **in that** the dialysis machine has a substitution line which preferably leads into the venous line of the extracorporeal blood circuit (16), with the control unit being configured such that the fluid volume withdrawn in the conditioning phase in the dialyzer (15) is at least partly substituted using this line, preferably after the conditioning phase and in particular during a flushing phase

2. A dialysis machine in accordance with claim 1, **characterized in that** the conditioning cycle is carried out at the start of the treatment.

3. A dialysis machine in accordance with one of the preceding claims, **characterized in that** the ratio of the ultrafiltration rate to the blood flow rate caused by the blood pump amounts to between 1.5 to 1 and 2.5 to 1, preferably between 1.8 to 1 and 2.2 to 1, and preferably approximately 2 to 1.

4. A dialysis machine in accordance with one of the preceding claims, **characterized in that** little or no dialysate flows into the dialysate side of the dialyzer (15) during the conditioning phase.

5. A dialysis machine in accordance with one of the preceding claims, **characterized in that** the duration of the conditioning phase amounts to 15 to 120 seconds, and preferably 30 to 60 seconds.

6. A dialysis machine in accordance with one of the preceding claims, **characterized in that** a fluid volume of between 20% and 70%, and preferably of between 30% and 60%, of the total volume of the blood side of the dialyzer (15) is withdrawn through the dialysis membrane during the conditioning phase.

7. A dialysis machine in accordance with one of the preceding claims, **characterized in that** the conditioning cycle is repeated, preferably at least 3x, and, for example, between 3x and 6x, with the repetitions preferably directly following one another.

8. A dialysis machine in accordance with one of the preceding claims, **characterized in that** the conditioning cycle comprises a flushing phase which follows the conditioning phase and in which the blood flow through the dialyzer (15) generated by the blood pump (18) exceeds the ultrafiltration rate.

9. A dialysis machine in accordance with claim 8, **characterized in that** the blood volume used in the flushing phase to flush the blood chamber of the dialyzer (15) amounts to 0.5 to 5 times, and preferably 1 to 2 times, the blood chamber volume.

## Revendications

1. Appareil de dialyse avec un circuit sanguin extracorporel, un dialyseur (15) et un circuit de dialysat (17), dans lequel, dans le circuit sanguin extracorporel (16), est disposée une pompe sanguine (18), dans lequel le dialyseur (15) comprend une membrane de dialyse qui sépare son côté sanguin de son côté de dialysat,
**caractérisé en ce que**
l'appareil de dialyse comprend une unité de commande qui est conçue pour effectuer un cycle de conditionnement qui comprend une phase de conditionnement dans laquelle un taux d'ultrafiltration dépasse le flux sanguin transporté par la pompe sanguine (28) à travers le dialyseur (15) et **en ce que** l'appareil de dialyse comprend une conduite de substitution qui conduit, de préférence dans la conduite veineuse du circuit sanguin extracorporel (16), dans lequel l'unité de commande est conçue de façon à ce que, à l'aide de cette conduite, le volume de liquide extrait du dialyseur (15) dans la phase de conditionnement soit au moins partiellement substitué, de préférence après la phase de conditionnement et plus particulièrement pendant une phase de rinçage.

2. Appareil de dialyse selon la revendication 1, **caractérisé en ce que** le cycle de conditionnement est effectué avec le début du traitement.

3. Appareil de dialyse selon l'une des revendications précédentes, **caractérisé en ce que** le rapport entre le taux d'ultrafiltration et le débit sanguin généré par la pompe est entre 1,5 à 1 et 2,5 à 1, de préférence entre 1,8 à 1 et 2,2 à 1 et de préférence environ 2 à 1.

4. Appareil de dialyse selon l'une des revendications précédentes, **caractérisé en ce que**, pendant la phase de conditionnement, un peu ou aucun dialysat ne s'écoule vers le côté de dialysat du dialyseur (15).

5. Appareil de dialyse selon l'une des revendications précédentes, **caractérisé en ce que** la durée de la phase de conditionnement est de 15 à 120 secondes et de préférence de 30 à 60 secondes.

6. Appareil de dialyse selon l'une des revendications précédentes, **caractérisé en ce que**, pendant la phase de conditionnement, un volume de liquide représentant entre 20 % et 70 % et de préférence entre 30 % et 60 %du volume total du côté sanguin du dialyseur (15) est extrait à travers la membrane de dialyse.

7. Appareil de dialyse selon l'une des revendications précédentes, **caractérisé en ce que** le cycle de conditionnement est répété de préférence au moins 3 fois et par exemple entre 3 fois et 6 fois, dans lequel les répétitions se succèdent de préférence immédiatement.

8. Appareil de dialyse selon l'une des revendications précédentes, **caractérisé en ce que** le cycle de conditionnement comprend une phase de rinçage qui succède à la phase de conditionnement et dans laquelle le flux sanguin généré par la pompe sanguine (18) à travers le dialyseur (15) dépasse le taux d'ultrafiltration.

9. Appareil de dialyse selon la revendication 8, **caractérisé en ce que** le volume de sang qui est utilisé dans la phase de rinçage afin de rincer la chambre sanguine du dialyseur (15) représente 0,5 à 5 fois et de préférence 1 à 2 fois le volume de chambre sanguine.
